# EUROPEAN PATENT SPECIFICATION

(11) **EP 2 604 264 B1**
(45) Date of publication and mention of the grant of the patent: **24.09.2014**
(21) Application number: 11851336.5
(22) Date of filing: 17.10.2011
(51) Int. Cl.: A61K 31/40, A61K 38/05, A61P 31/12

(54) **PHARMACEUTICAL COMPOSITION FOR TREATING VIRAL DISEASES**
PHARMAZEUTISCHE ZUSAMMENSETZUNG ZUR BEHANDLUNG VON VIRUSERKRANKUNGEN
COMPOSITION PHARMACEUTIQUE POUR TRAITER LES AFFECTIONS VIRALES

(30) Priority: 23.12.2010 RU 2010152865
(43) Date of publication of application: 19.06.2013
(73) Proprietor: Obshestvo S Ogranichennoj Otvetstvennostju "CytoNIR", St. Petersburg 191023 (RU)
(72) Inventor: SMIRNOV, Vjacheslav Sergeevich, St.Petersburg 197374 (RU)
(74) Representative: Jeck, Anton
(86) International application number: PCT/RU2011/000809
(87) International publication number: WO 2012/087181

(56) References cited:
- WO-A1-95/03067
- WO-A2-2005/112639
- RU-C1- 2 024 546
- RU-C2- 2 165 254
- CRISTINA FIORE ET AL: "Antiviral effects of Glycyrrhiza species", PHYTOTHERAPY RESEARCH, JOHN WILEY & SONS LTD. CHICHESTER, GB, vol. 22, no. 2, 1 February 2008 (2008-02-01), pages 141-148, XP002676791, ISSN: 0951-418X, DOI: 10.1002/PTR.2295 [retrieved on 2007-09-20]
- DATABASE WPI Week 200730 Thomson Scientific, London, GB; AN 2007-305604 XP002699432, -& JP 2007 070268 A (POLA CHEM IND INC) 22 March 2007 (2007-03-22)
- L. A. BALTINA ET AL.: 'Perspektivy sozdaniya novykh protivovirusnykh preparatov na osnove glitsirrizinovoi kisloty i ee proizvodnykh (obzor)' KHIMIKO-FARMATSEVTICHESKY ZHURNAL vol. 43, no. 10, 2009, pages 3 - 12
- 'Informatsiya o preparate. Istochnik informatsii: Spravochnik Vidal-2006' EPIGEN INTIM., [Online] Retrieved from the Internet: <URL:http://www.e-apteka.ru/doc/Vidal_docs/ drug_info_9585.asp?rfr=AptSite> [retrieved on 2012-01-12]
- V.V. IVANOVA ET AL.: 'Primenenie yantarnokislogo natriya v lechenii ostrykh respiratorno-virusnykh infektsy u detei' YANTARNAYA KISLOTA V MEDITSINE, PISCHEVOI PROMYSHLENNOSTI, SELSKOM KHOZYAISTVE. SBORNIK NAUCHNYKH STATEI. PUSCHINO vol. 300, 1996, pages 104 - 106
- KHAVINSON V.KH. ET AL.: 'Effect of Peptide Lys-Glu on Interleukin-2 Gene Expression in Lymphocytes' BULLETIN OF EXPERIMENTAL BIOLOGY AND MEDICINE vol. 130, no. 9, 2000, pages 898 - 899, XP055076189
- BALTINA L.A.: 'Sintez i otsenka biologicheskoi aktivnosti novykh konjugatov glitsirrizinovoi kisloty s aminokislotami i dipeptidami' BIOORG. KHIMIYA, [Online] vol. 35, no. 4, 2009, pages 563 - 571 Retrieved from the Internet: <URL:http://www.rjbc.ru/2009/4/abstracts/15 .shtml> [retrieved on 2012-01-19]

## Description

### Field of the Invention

The invention relates to medicine, in particular to pharmaceutical compositions based on peptides, in particular to the preparations used for treating viral diseases, such as influenza and acute viral respiratory disease (AVRD). These diseases, which are rather serious as they are, (especially influenza) are often followed by complications, such as pneumonia, bronchitis of various etiologies, inflammation of the maxillary or frontal sinus, etc.

### Prior Art

A medication for the treatment of influenza and AVRD, which shortens the duration of the illness and lowers the number of post infection complications, namely a recombinant (exogenous) interferon-alpha used in injection form with no less than 40,000 units twice a day for 10 days [N.G. Perminov et al. Reaferon. Koltzovo, 1993, p.p. 69 -71].

The disadvantage of this preparation is the risk of side effects, with chills, fever, headache, loss of weight, allergic reactions, leukopenia and thrombocytopenia, etc. being most common among them. In addition, administration of exogenous interferon may contribute to the formation of specific anti-interferon antibodies, which can completely neutralize the administered exogenous preparation. [Treatment with Interferon. WHO scientific group presentation. Series of technical presentations 676. Geneva. 1984. Hoshino. A., et al. // Antiviral Res. 1983, Vol. 3. P. 59-65].

Dipeptides, such as alpha-glutamyl tryptophan (a-glu-trp) and alpha-lysyl glutamine (α-lys-glu), are chemical compositions capable of inducing synthesis of endogenous interferon in patients. Endogenous interferon is more effective for the treatment of viral infections than exogenous interferon because administration of an interferon inducer does not promote excessive interferon formation and thus does not cause side effects due to the possible overdose and in addition, the patient's body does not form anti-interferon antibodies to foreign interferon Peptide inducers are safe for therapeutic use. [V.G. Morozov et al. Peptide Thyromimetics. Spb Nauka, 2000, p.p. 19, 33].

A-glu-trp can restore the immunity indicators that had been reduced by myxovirus (causing influenza) infections [V.S. Smirnov, A.A. Selivanov, Prevention and Treatment of Influenza Regulators. SPb. Nauka,1996]. Its preventive effect lasts no more than 2 months, after which the susceptibility to the virus is not only restored but can even increase. With the already existing infection, α-glu-trp administration does make a significant impact on the course and the outcome of the pathological process. Biological properties of γ-glu-trp dipeptide are similar to those of a-glu-trp and it is as effective as a-glu-trp [Li KaT Wa T.G. et al/ Brit.J.Clin.Pharmacol., 1996, 42, p.365-370].

A-glu-trp dipeptide in the thymosin-alpha molecule [J. Caldarella et al. Proc.Nati.Acad. Sei. USA, 1983, 80, p.7424-7427] also exhibits immunomodulating activity.

The pharmaceutical composition for parenteral administration comprising 0.001 - 0.01 % γ-glu-trp by weight as an acetate and a pharmaceutically acceptable additive such as 5 % aqueous glycine solution, 6 % saline polyglucin solution, etc. [RU patent #2120298, M. cl. A61κ 38/05, 1998] was used to correct immunodeficiency in patients, although it is hardly effective for the treatment of viral diseases such as influenza and AVRD.

The pharmaceutical composition "Citovir-3" comprising 0.001 - 0.01 % dipeptide by weight as well as 2-benzyl benzimidazole hydrochloride and ascorbic acid [RU patent #2165254, M.cl. A61K, 1991] is used for the prevention and treatment of influenza and AVRD. The composition comprises a monosodium a-glu-trp salt as the dipeptide promoting the endogenous induction of interferon-alpha, which is most effective at the onset of a viral infection. 2-benzyl benzimidazole hydrochloride, which is an interferon's co-inductor, maintains the immunostimulating function of α-glu-trp. Ascorbic acid at 0.02 - 0.050 g per dose is a substrate of the terminal link of the mitochondrial breathing complex and it enhances the effect of the first two components.

Citovir-3 was shown to be highly effective as a preventive treatment during AVRD and influenza epidemics. [K.S. Shipitsin et al. Epidemiological Infectious Diseases. 2010. #1, p.p. 57 - 61]. However, if Citovir-3 was administered after the clinical onset of the disease, its efficacy was much lower. In addition, elevated doses of ascorbic acid may promote acute gastritis and, in rare cases, renal colic in patients suffering from kidney stone disease.

### Disclosure of the Invention

The technical result at which the claimed invention is directed is to create a long-term antiviral resistance that is required and sufficient for a quick and effective elimination of the virus and the removal of the symptoms of the disease.

Said technical result is achieved with a pharmaceutical composition comprising an endogenous interferon inducer as a glutamic acid-based dipeptide and an endogenous interferon co-inducer, the latter comprising glycyrrhizic acid as a sodium or ammonium salt, and the dipeptide is selected from the group comprising α-glu-trp, γ-glu-trp, and α-lys-glu in the following component ratio (mg per dose):

| | |
|---|---|
| Glutamic acid-based dipeptide | 0.01 - 0.03 |
| Glycyrrhizic acid | 10 - 500 |

The pharmaceutical composition may further comprise 50 - 500 mg/dose of sodium fumarate or sodium succinate as the compound enhancing the effect of the first two components.

Glycyrrhizic acid C₄₂H₆₂O₁₆ is a triterpene compound with the following general formula:

| |
|---|
| 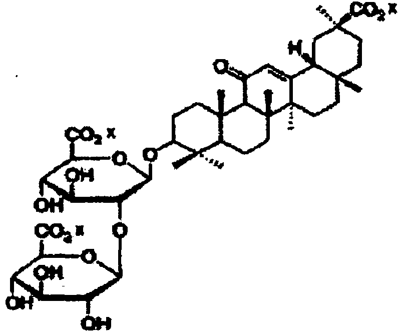 |
| where X = H, Na, NH₄ |

isolated from glycyrrhizin glycoside obtained from licorice root.

Glycyrrhizic acid exhibits antiviral activity against herpes and papilloma viruses [G.A. Tolstikov et al. Licorice:Biodiversity, Chemistry, Application in Medicine. Novosibirsk, "Geo," 2007, p.p. 305 - 207]. Glycyrrhizic acid-containing pharmaceutical composition "Phosphogliv," used in the complex hepatitis C therapy, was also described [O.M. Ipatova, Phosphogliv: Mechanism of Action and Clinical Application. M., 2005, p. 318].

Glycyrrhizic acid is known to reduce morbidity and mortality in animals infected with a lethal dose of influenza virus [Utsunomiya T. et al. Antimicrob. Agents Chemother., 1997, v.4, N 3, p.551-556], however, our data showed that the efficacy of glycyrrhizic acid administered to animals with already advanced infection was quite low.

A compound in one pharmaceutical composition comprising glycyrrhizic acid and glutamic acid-based dipeptide, in particular alpha-glutamyl tryptophan (thymogen), was problematic for the following reason:

The structure of glycyrrhizic acid is close to that of corticosteroids, i.e. to the adrenal hormones. Thymogen is a functional analogue to thymus hormone. Thymus and adrenal hormones are known to be antagonistic to each other. Thus, thymalinum (thymus extraction) administration was shown to significantly reduce cortisol's concentration [G.M. Yakovlev et al. Bioregulatory Mechanisms. SPB. "Nauka." 1992, p. 20]. Thus, the absence of antagonism between said compounds and the expressed summation of the therapeutic actions of the composition comprising these compounds was unexpected and unpredicted from the known properties of the components.

Glycyrrhizic acid is poorly soluble in water and gastric fluid. Therefore it was used as a trisodium or triammonium salt (they have identical properties), which are readily soluble in water and in gastric fluid.

The claimed pharmaceutical composition is intended for oral use and can be manufactured in tablet, capsule, or syrup form.

The components of the composition do not chemically react with each other; their composition can be prepared by mixing in common pharmaceutical equipment followed by tableting or encapsulating and then packaging. The composition may contain common pharmaceutically acceptable additives.

The efficacy of the claimed composition was studied on a survival model of the animals infected with a lethal dose of A/H3N2/ influenza virus who were administered a 5-day oral course of the claimed composition.

The animals used in the experiment were outbred male mice weighing 16 - 18 g obtained from the Rappolovo nursery of the Russian Academy of Medical Science. For one week prior to the experiment, the animals were held in a vivarium in isolation. All animals were kept on a balanced diet under standardized temperature and humidity.

Each experimental group consisted of 30 animals.

To simulate the experimental influenza infection, A/H3N2/Puerto-Rico-8/34 virus strain adapted for mice was attenuated via passage through embryonated eggs. Pre-challenge viral titer was 10³-10⁴ LD₅₀/ml. The animals were infected intranasally. Viral doses were 5 LD₅₀/ml and 10 LD₅₀/ml for the two sets of groups.

The survival of mice in the control groups on the 10^{th} day post challenge with said doses was zero, i.e. the examined doses were lethal.

The treatment of mice with the claimed composition was started 24 hrs. post challenge. The compositions were administered intraperitoneally via a special atraumatic metal tube in an isotonic sodium chloride solution. The medication dose was calculated per 1 kg of the animal's weight. The volume of the solution was 0.2 ml. The treatment lasted 5 days. The efficacy of the composition was determined by the survival of the animals on the 21^{st} day post challenge.

The test results are presented in the table. For comparison, the data on the efficacy of the separate composition components are also listed in the table: glycyrrhizic acid (row 8), a-glu-trp (row 9), and α-lys-glu (row 10).

The experiment demonstrated that when the composition additionally contained sodium fumarate or sodium succinate, the level of myeloperoxidase in blood serum was significantly higher than that in the animals receiving the two-component composition, which is explained by the reduced singlet oxygen production and the reduction in the ensuing cell damage.

The table demonstrates that separate components of the composition, both glycyrrhizic acid and the dipeptides are not sufficient and hardly effective for the treatment of animals infected with a lethal dose of the virus.

The treatment of influenza with the claimed composition ensures survival of the animals even when they are infected with the dose (LD₅₀ = 10) exceeding the lethal dose (LD₅₀ = 5) of the virus twofold.

**Table**

| Survival of Animals who have been Administered a 5-Day Oral Course of the Claimed Composition | | | | | | | |
|---|---|---|---|---|---|---|---|
| Content of the pharmaceutical composition mg/dose | | | | | | Viral dose LD₅₀ | Survival on 21^{st} day |
| Row | α-glu-trp | γ-glu-trp | α-lys-glu | glycyrrhizic acid Na salt | glycyrrhizic acid NH4 salt | | |
| 1 | - | - | 0.01 | - | 100 | 5 | 100 |
| | | | | | | 10 | 83.3 |
| 2 | - | - | 0.03 | 100 | - | 5 | 100 |
| | | | | | | 10 | 80 |
| 3 | 0.01 | - | - | 10 | - | 5 | 100 |
| | | | | | | 10 | 83.3 |
| 4 | - | 0.01 | - | - | 10 | 5 | 100 |
| | | | | | | 10 | 80 |
| 5 | - | - | 0.01 | - | 500 | 5 | 100 |
| | | | | | | 10 | 83.3 |
| 6 | 0.01* | | | | 10 | 5 | 100 |
| | | | | | | 10 | 93.3 |
| 7 | 0.01** | | | 10 | | 5 | 100 |
| | | | | | | 10 | 90 |
| 8 | - | - | - | 100 | - | 5 | 30 |
| | | | | | | 10 | 30 |
| 9 | 0.01 | - | - | - | - | 5 | 10 |
| | | | | | | 10 | 10 |
| 10 | - | - | 0.01 | - | - | 5 | 20 |
| | | | | | | 10 | 10 |

| | | | | | | | |
|---|---|---|---|---|---|---|---|
| * The composition also comprised 500 mg of sodium fumarate ** The composition also comprised 50 mg of sodium succinate | | | | | | | |

### Industrial Applicability

The pharmaceutical composition for the treatment of viral diseases may be manufactured at pharmaceutical plants specializing in oral medications.

## Claims

1. A pharmaceutical composition for the treatment of viral diseases comprising an endogenous Interferon inducer as a glutamic add-based dipeptide and an endogenous interferon co-inducer, wherein said endogenous interferon co-inducer comprises glycyrrhizic acid as a sodium or ammonium salt, and said dipeptide is selected from the group comprising alpha-glutamyl tryptophan, gamma-glutamyl tryptophan, and alpha-lysyl glutamine in the following component ratio (mg per dose):
| | |
|---|---|
| Glutamic acid-based dipeptide | 0.01 - 0.03 |
| Glycyrrhizic acid | 10 - 500.0 |

2. The pharmaceutical composition of claim 1, wherein said composition additionally comprises sodium fumarate in the amount of 50 - 500 mg per dose.

3. The pharmaceutical composition of claim 1, wherein said composition additionally comprises sodium succinate in the amount of 50 - 500 mg per dose.

## Patentansprüche

1. Pharmazeutische Zusammensetzung zur Behandlung von Viruskrankheiten, das aus einem endogenen Interferon-Induzierer als einem auf Glutaminsäure basierenden Dipeptid und aus einem endogenen Interferon-Koinduzierer besteht, wobei der endogene Interferon-Koinduzierer aus Glycyrrizinsäure als ein Natrium- oder Ammoniumsalz besteht, und das Dipeptid ausgewählt ist aus einer Gruppe, enthaltend alpha-Glutamyltryptophan, gamma-Glutamyltryptophan, und alpha-Lysylglutamin, in folgendem Komponentenverhältnis (mg pro Dosis)
| | |
|---|---|
| auf Glutaminsäure basierendes Dipeptid | 0.01 - 0.03 |
| Glycyrrizinsäure | 10 - 500.0. |

2. Die pharmazeutische Zusammensetzung nach Anspruch 1, wobei die Zusammensetzung zusätzlich Natriumfumarat in Menge von 50-500 mg pro Dosis enthält.

3. Die pharmazeutische Zusammensetzung nach Anspruch 1, wobei die Zusammensetzung zusätzlich Natriumsuccinat in Menge von 50 - 500 mg pro Dosis enthält.

## Revendications

1. Composition pharmaceutique pour le traitement de maladies virales, comprenant un inducteur d'interféron endogène en tant que dipeptide à base d'acide glutamique et un co-inducteur d'interféron endogène, ledit co-inducteur d'interféron endogène comprenant de l'acide glycyrrhizique en tant que sel d'ammonium ou de sodium, ledit dipeptide étant choisi parmi le groupe comprenant l'alpha-glutamyl-tryptophane, le gamma-glutamyl-tryptophane et l'alpha-lysyl-glutamine dans le rapport de composants suivant (mg par dose) :
| | |
|---|---|
| dipeptide à base d'acide glutamique | 0,01 - 0,03 |
| acide glycyrrhizique | 10 - 500,0. |

2. Composition pharmaceutique selon la revendication 1, dans laquelle ladite composition comprend en outre du fumarate de sodium en quantité de 50 à 500 mg par dose.

3. Composition pharmaceutique selon la revendication 1, dans laquelle ladite composition comprend en outre du succinate de sodium en quantité de 50 à 500 mg par dose.
